Europäisches Patentamt

European Patent Office

Office européen des brevets

(11) Publication number: 0 270 206 A1

# (12) EUROPEAN PATENT APPLICATION

(21) Application number: 87307587.3

(22) Date of filing: 27.08.87

(51) Int. Cl.⁴: G01N 33/543

(30) Priority: 29.08.86 GB 8620893

(43) Date of publication of application:
08.06.88 Bulletin 88/23

(84) Designated Contracting States:
AT BE CH DE ES FR GB GR IT LI NL SE

(71) Applicant: UNILEVER PLC
Unilever House Blackfriars P.O. Box 68
London EC4P 4BQ(GB)

(84) GB

(71) Applicant: UNILEVER NV
Burgemeester s'Jacobplein 1 P.O. Box 760
NL-3000 DK Rotterdam(NL)

(84) BE CH DE ES FR GR IT LI NL SE AT

(72) Inventor: Davis, Paul James
The Hawthorns Pavenham Road
Bedfordshire MK43 7EX(GB)
Inventor: Drake, Rosemary Ann Lucy
1 Warren Cottages Church End
Barley Royston Hertfordshire SG8 8JP(GB)
Inventor: Hornby, William Edward
1 Ray Mill Road West
Maidenhead Berkshire SL6 8FA(GB)

(74) Representative: Butler, David John et al
Unilever PLC Patents Division P.O. Box 68
Unilever House
London EC4P 4BQ(GB)

(54) Assays.

(57) An immunoassay for determining qualitatively or quantitatively the presence of an analyte in a sample by means of specific binding, wherein the sample is contacted with a moveable solid phase carrier material, e.g. particles (21) on which is immobilised a first binding reagent having specificity for the analyte and with a labelled reagent which can participate in either a "sandwich" or a "competition" reaction with the first reagent in the presence of the analyte and, following an incubation period sufficient to allow the reaction to take place, the carrier material is moved within the assay medium (20) to a location adjacent a signal sensing means (19), in the immediate vicinity of which a signal influencing agent, e.g. a substrate, is released into the assay medium and cooperates with the label in the generation of a signal, such as chemiluminescent light, the magnitude of the signal generated in the vicinity of the sensing means being used as a measure of the extent to which the binding reaction has occurred. Preferably the assay medium incorporates a masking or quenching agent which suppresses any signal generated in regions of the assay medium remote from the signal sensing means.

Fig. 1

## ASSAYS

The present invention relates to assays utilising specific binding, such as immunoassays.

Solid phase, e.g. particle-based, immunoassay systems are already known. These involve the qualitative and/or quantitative determination of an immunogenic species in a test sample by means of an immunological binding reaction between the immunogenic species (analyte) and one or more specific binding partners therefor, at least one of which binding partners is linked to an insoluble carrier material in contact with a liquid medium comprising the test sample. The fact that the resulting immunological complex is linked to the carrier material can be used to advantage during the subsequent determination of the extent to which the binding reaction has taken place. The determination is usually achieved by the use of a labelled specific binding partner, and the binding reaction can be either a "competition" reaction or a "sandwich" reaction, both of which are now widely used in this art.

In a typical "competition" reaction, the assay medium, which is generally aqueous, is contacted with the solid phase carrier on which is immobilised a specific binding reagent (e.g. monoclonal antibody) having specificity for the analyte to be determined, and the assay medium contains a known quantity of the analyte (or an analogue thereof possessing the identical epitopes) bearing a label and a known quantity of a sample suspected of containing the analyte. In the absence of analyte in the sample, the labelled analyte or analogue binds with the specific binding reagent immobilised on the solid phase carrier and the extent of this binding is determined from standard experiments. When the sample contains the analyte, this competes with the labelled analyte or analogue for the specific binding reagent and hence the extent to which the specific binding reagent becomes coupled to the labelled component is reduced. By comparing this with the standard situation, (i.e. the result obtainable in the absence of the analyte) a measure of the analyte concentration in the sample can be derived.

In a typical "sandwich" reaction, a first binding reagent having specificity for the analyte is immobilised on the solid phase carrier, and the assay medium, which is again generally aqueous, also contains a second binding reagent having specificity the analyte and which bears a label. In the absence of analyte, no coupling can occur between the immobilised reagent and the labelled reagent. When the analyte is present it acts as a bridge between the immobilised and labelled specific binding reagents and results in indirect coupling of the labelled reagent to the solid phase. The extent of this coupling provides a measure of the analyte concentration in the sample. The specificities of the immobilised reagent and the labelled reagent for the analyte can be different, but, if the analyte molecule possesses more than one identical epitopes, it is possible to use the same specific binding reagent both in the immobilised form and in the free-labelled form.

Several types of particle-based immunoassay systems are already known. Some of these involve complicated detection systems for the complex that results from the specific binding reaction, and/or the complete physical separation of the particulate carrier material carrying the complex from the test medium, including removal of all unbound material, prior to determination of the species under test. In one embodiment, the present invention seeks to provide a non-separation particle-based immunoassay systems in which the determination of the species under test can be made rapidly and simply, and especially immunoassays wherein only one step is required.

Immunoassays involving the localisation of particulate solid phase carriers within an assay medium are described in various documents. EP 0169434 describes a fluorometric immunological assay method in which an antigen is attached to magnetic particles which are, for the time of measurement, pulled against the wall of a measurement vessel by means of a magnetic field. To obtain an analytical result in such a system, it is, of course, necessary to stimulate the fluorescence by the external application of energy such UV light.

EP 0149565 describe immunoassays performed using a labelled reagent and another reagent bound to magnetically attractable particles, which are suspendable but insoluble in a liquid assay medium. After the labelled reagent has become partitioned between the liquid phase and the particles, in proportions which depend on the concentration of an analyte in a sample, the liquid phase is removed. Then the particles are resuspended in another liquid medium and the concentration of label observed. The method is said to be particularly suitable for fluorescent and chemiluminescent label systems, and can conveniently be performed in microtiter plates in which the wells are optically screened from one another, the observation being made from above or below the wells. EP 0149565 specifically states that it is difficult to make useful measurements of any optical signal generated from the pellet which results from bringing down a particulate reagent out of suspension.

The philosophy behind the invention described in EP 0149565 is completely opposite to that on which the present invention is based, because we have found that not merely is it possible but, indeed, it is very advantageous to make the relevant observations from the localised solid phase rather than from the dispersed solid phase.

The invention provides a carrier-based assay system which utilises a specific binding partner which is labelled such that the label can participate in a chemical reaction to provide a chemical "signal", and in which system, following incubation with a test sample to provide the specific binding reaction, the carrier material is moved through the assay medium to the immediate vicinity of a sensing means for the chemical "signal" and in the immediate vicinity of which sensing means a signal influencing agent is released into the test medium, so enabling the generation of a detectable signal the nature and/or magnitude of which is dependent on the extent of binding that has taken place between the immunogenic species under test and the specific binding partner carried by the particles.

More specifically, the invention provides an assay method for determining qualitatively and/or quantitatively the presence of an analyte in a sample by means of specific binding, wherein the sample is contacted with a moveable solid phase carrier material on which is immobilised a first binding reagent having specificity for the analyte and with a labelled specific binding reagent which can participate in either a "sandwich" or a "competition" reaction with the first reagent in the presence of the analyte, and in which method, following an incubation period sufficient to allow the reaction to take place, the moveable solid phase carrier material is moved within the assay medium to the immediate vicinity of a sensing means for the chemical "signal" and in the immediate vicinity of which sensing means a signal influencing agent is released into the test medium, so enabling the generation of a detectable signal the nature and/or magnitude of which is dependent on the extent of binding that has taken place between the immunogenic species under test and the specific binding partner carried by the particles.

The signal influencing agent can be a chemical entity that forms an essential part of the signal generating chemical reaction. In this embodiment, for example the chemical "signal" which is potentially generated by the label attached to the specific binding partner, will not be produced until the label encounters the signal influencing agent.

Alternatively, the signal influencing agent can be a chemical entity that enhances the efficiency of the chemical reaction by which the chemical "signal" is produced, and hence which magnifies the extent of the signal production when the labelled specific binding partner is in the immediate vicinity of the sensing means.

A concentration gradient of the signal influencing agent will be established in the assay medium with the highest concentration of the signal influencing agent remaining in the immediate vicinity of the sensing means. If the signal sensing activity requires only a brief period of time, an effective concentration gradient will be established even in a very small volume of test sample because the sensing time will be insufficient for diffusion to cause a high concentration of the signal influencing agent throughout the body of the assay medium. An optional feature of the invention involves the incubation of a small volume of test sample, followed by dilution of the sample prior to localisation of the carrier material of the vicinity of the sensing means. The greatly increased volume of the diluted sample will assist the establishment of a suitable concentration gradient of the signal enhancing agent.

The necessary local release of the signal influencing agent can be achieved, for example, by allowing the signal influencing agent to diffuse from a source located on, in or near the signal sensing means. For example, if the signal influencing agent can exist as a water soluble solid, a quantity of the solid form fixed on, in or near the sensing means can be allowed to dissolve into the liquid assay medium so providing a concentrated solution of the signal influencing agent in the immediate vicinity of the sensing means. Alternatively, the signal influencing agent can be encapsulated or similarly physically restrained in, on or near the signal sensing means such that in contact with the liquid assay medium the signal influencing agent is released slowly into the assay medium. For example, the signal influencing agent can be retained in a layer or body of gel comprising, covering or located adjacent to the signal sensing means and from which the signal influencing agent can escape into the liquid assay medium.

An assay test kit in accordance with the invention can therefore include a vessel in which the assay can be conducted, incorporating the signal sensing means and a source of the signal influencing agent located in, on or near the signal sensing means. For example, a signal influencing agent capable of existing in a water soluble solid form can be coated as the solid form onto the inner surface of the signal sensing means and/or the inner wall of the vessel in the immediate vicinity of the sensing means. Alternatively the signal influencing agent in releasably encapsulated form can be bound in, on or near the sensing means.

The net effect is that the generation of the chemical signal in the immediate vicinity of the sensing means will be enhanced, but the complete (or relatively low) absence of the signal influencing agent in regions of the test medium that are remote from the sensing means will minimise any "background" chemical signal originating from labelled specific binding partners that have not participated in the binding reaction and which are therefore free in the bulk of the assay medium.

The possibility of a background signal can be further reduced by incorporating a masking or quenching agent in the assay medium which inhibits the production or observation of the chemical signal. The masking or quenching agent should uniformly dispersed within the bulk of the assay medium, but its concentration should be chosen such that following localisation of the carrier material in the immediate vicinity of the sensing means, and local release of the signal influencing agent, the influence of the masking or quenching agent in the immediate vicinity of the signal sensing means is insufficient to prevent the generation of a detectable signal. The signal suppressing effect in the bulk sample must be balanced such that a local concentration of the label produces a detectable signal. In any given system, the level of suppression has to be determined by experiment and experience. However, this is not difficult to achieve. A dispersed mixture of the carrier material, bound signal generator and free signal generator, representing the concentration that would be expected in the non-localised sample is, "titrated" with the appropriate signal suppressing means (e.g. a consumer reagent) until the level of signal suppression is just sufficient to ensure that there is no net production of signal in the absence of localisation of the bound signal generator.

The invention will be described in detail principally in the context of particle-based immunoassays, but from the following description it will become apparent that other practical applications can be effected without departing from the broad spirit of the invention.

In the context of the present invention, the term "signal" is used to denote any phenomenon that can be observed or measured, qualitatively or quantitatively, and the term "detectable signal" is used to denote any significant change in the signal that can be observed or measured. For example, the signal can be the concentration of a chemical entity, and the detectable signal can be an increase or decrease in concentration from a relatively uniform "background" signal (which might be zero). Another example of a detectable signal would be a change in the rate of accumulation of a chemical entity. A further example of a detectable signal would be change in the intensity of electromagnetic radiation, such as visible light, emitted by a chemical species such as a chemiluminescent agent.

An immunoassay system of the invention is therefore characterised by the features:

a. the label has the potential to generate a signal continuously while in the assay medium if the medium contains appropriate co-reagents;

b. complexing of the labelled binding partner with the other binding partner does not of itself significantly alter the signal generating potential of the label; and

c. a detectable signal is produced only when the carrier material, to which is linked the complexed label, is localised in a comparatively small zone within the assay medium, adjacent the signal sensing means, into which zone a signal influencing agent is released.

The solid phase, e.g. the particulate carrier, initially has access to the bulk of the assay medium, and hence can be under the influence of any signal-suppressing activity that the bulk medium possesses. The signal suppressing activity of the bulk medium, may simply be a dilution effect or may be supplemented by the presence of one or more chemical of physical agents that interfere with the production of a signal. Subsequently, the solid phase is placed in a situation adjacent a signal sensing means where it has relative freedom from the bulk sample and hence is less influenced by the signal-suppressing effect. The simple act of physically localising the carrier particles is all that is required in order for a detectable signal to be produced.

The suppression of any signal generated by the labelled binding partner when dispersed in the test medium is a desirable preferred feature of the invention. The mechanism by which the signal is suppressed will depend on the nature of the signal generating system itself. The following mechanisms, which are given by way of example only, illustrate the range of possibilities that exist.

The label can be a chemical agent that reacts with a substrate to produce a chemical product. If the test medium contains in appropriate concentration a consumer reagent that destroys all or most of the chemical product as soon as it is formed, a uniform background signal (e.g. the absolute concentration or rate of change of concentration of the chemical product) will be maintained as long as the label remains dispersed throughout the bulk of the assay medium. However, following a binding reaction with a specific binding partner linked to the carrier particles, localisation of the particles within a zone in the assay medium adjacent a signal sensing means and into which zone the necessary substrate is being released into the medium, can cause the chemical product to accumulate at a rate which significantly exceeds the rate at which the consumer reagent can destroy the chemical product in that zone. The increasing quantity of chemical product in the immediate vicinity of the localised particles will constitute a detectable signal.

While the particulate carrier material is localised within a zone in the assay medium, the consumer reagent must remain uniformly dispersed throughout the bulk of the assay medium.

Such a chemical suppression can be achieved easily using, for example, glucose oxidase enzyme as the label, glucose as the releasable substrate and catalase enzyme in the test medium as the consumer reagent. The concentration of catalase in the assay medium can be chosen such that under normal circumstances, while the glucose oxidase is uniformly dispersed in the assay medium, all or most of the hydrogen peroxide produced by any reaction between the glucose oxidase and the glucose is immediately destroyed by the catalase. The background level of hydrogen peroxide will remain constant or will alter only slowly and uniformly. However, when the particulate carrier bearing a complex including the glucose oxidase-labelled specific binding partner is concentrated in a comparatively small zone in the assay medium in the vicinity of the glucose-releasing sensing means, the amount of hydrogen peroxide accumulated in that locality will be sufficient to overcome the action of the catalase, and can be measured as an increased rate of accumulation of the peroxide. The hydrogen peroxide can be detected, for example, by using an appropriate electrode, the localisation of the particulate carrier being effected in the immediate vicinity of the electrode. Interfering chemicals, which can be present in the sample, such as ascorbic acid or paracetamol, and which can give rise to spurious electrochemical signals like that caused by peroxide, can be consumed by means of an electrode in the cell. This may avoid the need for selective membranes protecting the working electrode.

Alternatively the chemical product "signal" can be detected via a further chemical reaction with another reagent which is localised on or in a surface. For example, the particulate carrier material to which the complex is linked can be localised adjacent a surface (such as a slab, sheet, film or membrane, or a second particulate carrier material) containing or carrying a chemical reagent which interacts with the "signal" product and which results in, for example, the formation of a colour change in or on the surface. Taking as an example the hydrogen peroxide-generating system just described, the detector surface could contain or carry a peroxide-utilising enzyme, such as horseradish peroxidase (HRP), leading to the formation of a coloured product by oxidation of a substrate, such as tetramethylbenzide (TMB), in or on the detector surface.

Another alternative can be the use of a label that alters the pH of the assay medium, such as urease or penicillinase. A substrate for the label, e.g. urea in the case of urease, can be released into the assay medium in the vicinity of a pH sensing means. In the dispersed situation, the effect of the label can be masked or quenched by incorporating a pH buffer in the assay medium. Following the binding reaction and localisation of the particles carrying the complex, the local concentration of label will cause a pH change that overcomes the buffering capacity of the assay medium in the immediate vicinity of the signal sensing means. This pH change can be detected, for example, using a pH electrode or a pH-responsive indicator paper as the sensing means.

In a further alternative embodiment, the sensing means comprises a gas detector located outside the vessel in which the assay is conducted. The sensor can, for example, be a chemical field effect transistor (Chemfet), sensing ammonia. The sensor can be located adjacent a gas-permeable membrane in the vessel wall, against which the carrier material is localised. The label can be urease enzyme, converting locally-released substrate urea to ammonia, and the test medium can contain l-glutamate dehydrogenase as a consumer reagent.

A system that is completely different in detail but analogous in principle, can be made using a label which is chemiluminescent or which participates in a chemiluminescent reaction with one or more other components, at least one of which is released into the test medium in the vicinity of the sensing means. If the test medium is opaque or at least insufficiently translucent for the light to be observable when the labelled specific binding partner is evenly dispersed in the assay medium, an optical signal will not be detectable. Preferably however, the assay medium contains a chemical species that interacts with a component of the light-generating system, and quenches or inhibits the production of an optical signal. Subsequent localisation of the particulate carrier to which is linked a complex involving the labelled specific binding partner, adjacent the sensing means, can give rise to a localised optical signal which is of sufficient intensity to be detectable. The localisation can be effected, for example, adjacent a "window" in the vessel holding the test medium, or adjacent an optical sensor. The optical sensor can be, for example, a photomultiplier tube. Alternatively, the light can be recorded on a photographic film, e.g. using a "camera" device such as is described in GB patent specification No. 2169704B.

The placing of a (second) optical sensor in the body of the test fluid to measure the background light intensity would be useful in providing means for calibration or normalisation.

Such a system can be provided using, for example, horseradish peroxidase as the label, with a peroxide (such as $H_2O_2$ in the medium, and luminol released into the medium in the immediate vicinity of a light detection means. An alternative system can involve glucose oxidase enzyme as the label, with luminol in the medium and glucose released into the medium in the immediate vicinity of a light detection means. The medium can optionally contain a dye, sufficiently chemically inert not to be affected by the peroxide reaction, which gives the medium an optical density at an appropriate wavelength that is sufficiently high (preferably O.D. of at least about two) to render any light generated by the dispersed label insignificant in comparison with that observable from a localised concentration of the label in the vicinity of the detection means. Most preferably, however, the assay medium contains a chemical reagent that interferes with the generation of the luminescence.

An alternative system can involve a competing reagent, such as gallic acid or thiodiglycollic acid, in the assay medium, horseradish peroxidase as the label reacting with hydrogen peroxide and luminol to produce visible light, the luminol being released into solution in the immediate vicinity of the light sensing means.

A further alternative will involve a detection zone, such as a gel layer, containing luminol, bound HRP and releasable glucose as substrate, the label being glucose oxidase.

Although luminol (5-amino-2,3-dihydro-1,4-phthalazinedione) is a particularly preferred chemiluminescent agent for use in the context of the invention, a range of alternative compounds and reactions can be used. For example, other derivatives of 2,3-dihydro-1,4-phthalazinedione, such as the 6-amino derivative (isoluminol) can be used. Other examples are luciferin and acridinium esters. Such systems are generally described, for example, in GB 1552607, GB 2112779A and GB 2095830B. The enhancement of chemiluminescent reactions, using compounds such as p-iodophenol, is described in EP 87959, EP 116454 and GB 2162946A.

An important embodiment of the invention is a particle-based immunoassay system which utilises a specific binding partner which is labelled with a reagent that participates in a chemiluminescent reaction, one or more other essential reagent(s) for the chemiluminescent reaction being released into the assay medium in the immediate vicinity of a light detection means, any light produced being masked or quenched while the labelled binding partner is uniformly dispersed within the test medium, and in which system following the binding reaction a local concentration of the particulate carrier material is established in the assay medium adjacent the detection means such that the local concentration of complexed labelled binding partner carried by the particles provides a local emission of light which is sufficient to overcome the local masking or quenching ability of the test medium and hence to provide a detectable signal, the nature and or magnitude of which is dependent on the extent of binding that has taken place between the species under test and the specific binding partner carried by the particles.

A further embodiment of the invention is a particle-based immunoassay which utilises:

(a) a first population of localisable particles (e.g. "magnetic" particles) bearing a specific binding partner for a chemical species under test;

(b) a non-particle-borne specific binding partner labelled with a chemical reactant that can react with a substrate to produce a precursor for a chemiluminescent reaction;

(c) optionally, a consumer reagent dispersed in the assay medium which competes effectively for the precursor and can thereby inhibits the production of chemiluminescence while the labelled specific binding partner remains dispersed in the assay medium;

(d) a second population of localisable particles bearing or containing a reagent which can react with the precursor to generate chemiluminescence;

(e) a light detection means; and

(f) a source of substrate for the chemiluminescent reaction which can release the substrate into the medium in the immediate vicinity of the light detection means, and in which assay co-localisation of the two particle populations adjacent the light detection means enables observation of chemiluminescence produced in an amount dependent on the extent to which the specific binding partner carried by the first population of particles has bound to the species under test.

In the foregoing embodiment, the label can be, for example, glucose oxidase reacting with glucose released into the medium in the immediate vicinity of the light detection means, and with oxygen in the test medium to produce hydrogen peroxide as the precursor, the consumer can be catalase enzyme, and the bound reagent can be horseradish peroxidase interacting with luminol in the text medium. If desired, the bound reagent can be carried on the surface or entrapped within the particles of the second population.

Sequential addition of the components in the test medium can be advantageous, e.g. to permit adequate time ("incubation") for the immunological binding reaction(s) to take place prior to the addition of a substrate required for the generation of a signal by the label. It will thus be appreciated that the continuous generation of signal by the label will only take place when the necessary substrates have been added. This addition will always be made before the solid phase is relocated to the detection zone or environment. The continuity of the signal generation must last at least for the duration of the time taken to localise the solid phase and to detect the signal produced therefrom.

The incubation period needed for the specific binding reaction(s) to take place will depend on many factors, such as the nature of the reagents and the analyte, their relative and absolute concentrations, and on physical factors such as temperature. These parameters are familiar to those skilled in this art. In general, the incubation period will not exceed about an hour, and for most applications will lie in the range 30 minutes to one hour.

Localisation of the carrier material can be achieved in a wide variety of ways.

One simple embodiment is to allow particles to settle under gravity, although this necessitates a uniform suspension of the particles in the test medium being maintained, eg. by stirring, while the binding reaction is taking place. Centrifugation can be used as an alternative to gravitation. Alternatively, particles can be localised by passing a porous membrane or filter through the bulk of the test medium, thus sweeping the particles into a small volume. Another alternative is to use a particulate carrier material which, under the influence of magnetism or another externally-applied force, can be urged into a particular locality within the bulk of the test medium. The particles can be moved through a test medium by the ultrasonic generation of a drifting standing wave.

The solid phase can be made of any suitable carrier material. So-called "magnetic" (i.e. particles that can be induced to move through a liquid under the influence of a magnetic field) particles of a variety of suitable sizes, ranging for example from less than about 1 to more than about 300μ, are available commercially. For gravity separation, particle sizes of at least about 50μ, and not greater than about 300μ, are preferred, depending on the density of the material. Commercially-available oxirane acrylic beads are very suitable, especially for assays involving labelled haptens. Techniques for sensitising and/or coupling such solid phase carriers to binding reagents, such as immunoglobulins, are well known standard techniques in the art, and form no part of the present invention.

The range of analytes to which the invention can be applied is vast. By way of example only, the following can be mentioned: Steroid hormones, such as those involved during pregnancy or which are influential on fertility, e.g. progesterone and oestrogens. Urinary metabolites such as oestrone-3-glucuronide and pregnanediol-3-glucuronide. Peptide hormones such as human chorionic gonadotrophin, luteinising hormone and follicle stimulating hormone. Proteins such as urinary albumin, beta-2 microglobulins and retinol binding protein. High and low-density lipoprotein, the detection of which is valuable in cardiovascular monitoring, and cardiac troponin. Infectious disease markers, such as Rubella antibodies and Toxoplasma antibodies. Immunoglobulins, such as monoclonal antibodies.

The test medium can thus be derived from many sources, especially body fluids such as urine, serum and plasma.

The specific binding partners can be polyclonal or monoclonal antibodies or antigens, lectins, or hormones and their receptors. Such materials are well known in the art, many are available commercially, and their precise nature forms no part of the present invention.

If the sample suspected of containing the analyte is of variable nature, (e.g. serum and urine) or contains components which could interfere with the specific binding reactions or with the production of the observable signal, it can be advantageous to dilute the sample, e.g. with water or a buffer solution, before an assay method of the invention is applied, to reduce the likelihood of such interference.

An assay test kit in accordance with the invention can comprise a re-usable cell, complete with the signal sensing means (e.g. electrodes), or a disposable cell incorporating a membrane through which the signal can pass to a re-usable sensing means, or a wholly disposable cell and signal sensing means combination. Appropriate reagents, e.g. the solid phase and other essential components, can also be supplied as necessary.

In one embodiment of the invention, a device for use in a method as hereinbefore set forth, comprises an open-topped vessel for containing a volume of assay medium, the vessel being closeable by means of a cap within which is located a body of water-insoluble gel incorporating a signal-influencing agent which is releasable from the gel when the gel is contacted with an assay medium, the gel also containing such further reagents as may be necessary to enable a detectable signal to be generated.

A further embodiment of the invention is an assay method which utilises a device as set forth in the immediately preceding paragraph, wherein an assay medium is prepared from:

    a) an aqueous sample;

    b) a particulate solid phase carrier material

which can readily separate under gravity from an aqueous medium, the solid phase carrier material bearing an immobilised first binding reagent having specificity for an analyte suspected of being present in the sample; and

c) a labelled specific binding reagent which can participate in either a "sandwich" or a "competition" reaction with the first reagent in the presence of the analyte; wherein a volume of the assay medium, either before or after an incubation period sufficient to allow the reaction to take place, is placed inside the device which is then closed with the cap and inverted to allow the particulate solid phase carrier material to settle onto the gel and enable an analytical result to be determined.

Various aspects of the invention are illustrated by the following Examples.

Example 1

The principle of the invention is illustrated by the following immunoassay test.

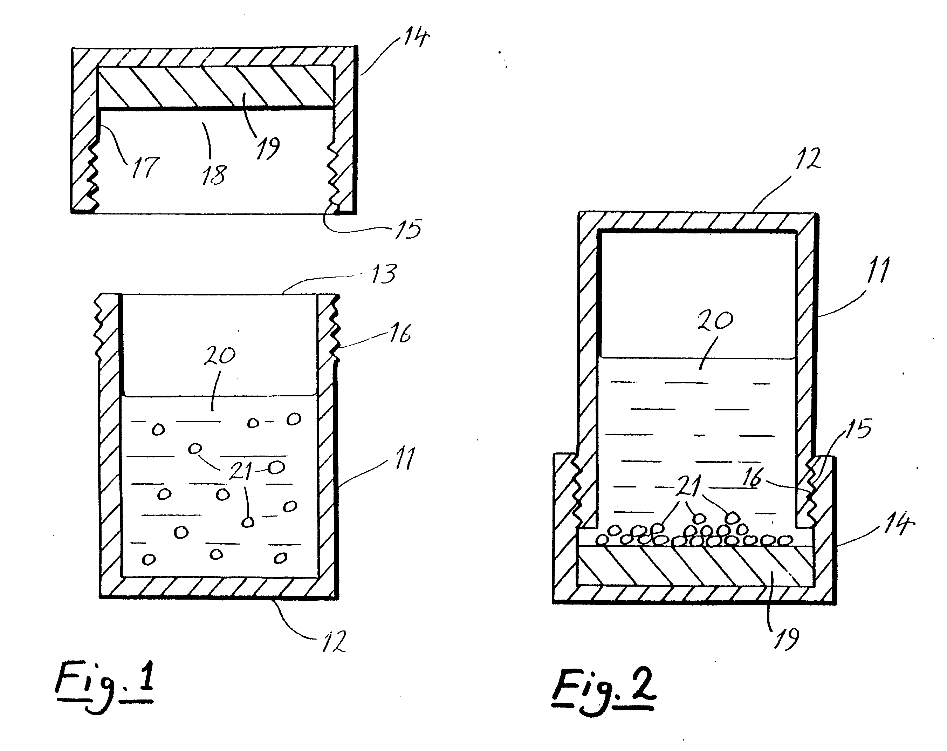

The apparatus comprising the test kit is illustrated in Figures 1 and 2 of the accompanying drawings.

Referring to Figure 1, the apparatus comprises a closable container formed from a cylindrical body 11 permanently closed of the bottom end 12 and open at the top end 13. Top end 13 is closable by means of a cap 14. Although this is not of significance to the invention, in this instance the cap is shown as having in internal diameter equal to the outer diameter of the body 11, and having an internally threaded portion 15 which engages with a correspondingly threaded outer portion 16 at the top of body 11 during closure. The internally threaded portion 15 of cap 14, extends only partially into the inside of the cap, leaving a deeper portion 17 of the inside wall as a smooth surface. The deeper volume 18 inside cap 14 bounded by wall portion 17 contains a gel layer 19 extending over the whole width of the cap. In Figure 1, the cap 14 and body 11 are shown separated one from another, and the body is partially filled with a liquid test sample 20 which includes dispersed therein a plurality of carrier particles 21.

Referring to Figure 2, this shows the body 11 closed by means of the cap 14 and inverted to allow the liquid sample 20 to come into contact with the gel 19. Figure 2 also shows that the carrier particles 21 are no longer dispersed in the liquid but have been allowed to settle onto the gel surface 22.

The carrier particles can participate in a particle-based immunoassay system as described in detail below. The gel acts as a sensing means in accordance with the invention and incorporates one or more components that result in detection of a chemical signal generated by means of a labelled partner that has become attached to the particles during the incubation step. The gel also acts a reservoir of the releasable signal influencing agent which, when the apparatus has been enclosed and inverted so the that liquid sample is in contact with the gel, can disperse from the gel into the liquid sample. When the particles bearing the labelled binding partner have accumulated on the surface of the gel, the chemical signal will be generated and can be detected on the gel surface or within the body of the gel if the chemical signal is such that it can diffuse into the gel mass. The chemical signal can be detected, for example, by causing a colour to be produced or changed within the gel. In order for the colour to be discernable without the need to remove the cap from the apparatus, it is preferable that the cap should be constructed wholly or partially from material that is transparent or at least translucent. For example, a transparent "window" can be provided in the upper surface of the cap or the side wall of the cap so that any colour change in the gel can be seen easily.

The pH in the gel can be different from that in the liquid test medium, thus allowing different enzymes to be used at their optimum pH's in the two environments.

If the gel is heat-settable, its setting temperature should not be so high that any delicate reagents, such as enzymes, held within the gel risk being denatured during manufacture of the assay kit. Preferably the setting temperature is at least 30°C, but preferably not greater than 40°C. Agarose gel is preferred because its melting point is ideal in this respect.

Using a test kit as just described, the following experiment was conducted.

Example 1a

A set of small closable transparent plastics sample tubes, were used as the basic assay cells. Each tube was about 1cm in diameter and about 3cm in height. 0.15ml of agarose detector gel was cast into the base of each tube.

Each tube was loaded with a liquid test sample comprising phosphate buffered saline (PBS) at pH 6, containing:

Solid phase

Commercially-available epoxy-activated oxirane acrylic beads (size range 100-200$\mu$m), sensitised with avidin from hen's eggs, at a level of 16mg avidin per 10g dry weight of beads. The avidin-linked beads were stored in phosphate-buffered saline solution (PBS) at pH 7.1.

### Conjugate

Biotinylated glucose oxidase was obtained commercially as a freeze dried powder and contained approximately 20nmols biotin per mg protein. It was reconstituted as recommended and diluted in PBST immediately before use.

### Biotin

d-biotin was obtained commercially as a crystalline solid, and solutions of appropriate concentration were prepared by dilution from a reference solution consisting of an accurately weighed quantity of biotin dissolved in PBS. The biotin concentration was varied, from 5ng/ml to 100 ng/ml.

A quantity of gel, based on 1.5% (weight by volume) agarose in PBS at pH 6.0, was prepared and contained 100mM glucose, 60mM potassium iodide, 1% (w/v) starch, and 0.005 units of encapsulated horseraddish peroxidase enzyme. The encapsulated HRP was obtained commercially and consisted of enzyme entrapped in polyamide micro capsules (20-80μ diameter) at a level of 0.656 units per mg of wet solids. The capsule membranes were semi-permeable, allowing free diffusion of small molecules such as hydrogen peroxide and potassium iodide but retaining molecules of molecular weight greater than 10,000.

Gel layers of this formulation were tested for their ability to detect peroxide by placing various solutions of differing peroxide concentration in a series of gel-containing tubes. It was found that 1ml of 50μM peroxide solution would produce a blue colour in the gel within 20 minutes.

### Assay Method

A sample of the avidin-sensitised beads was centrifuged and the supernatant removed. A 0.05% solution of keltrol in buffer solution was added to produce a 50/50 suspension of beads. A 5μg per ml solution of glucose oxidase biotin conjugate from the stock reagent was prepared in 0.05% keltrol in buffer. To each of a number of microfuge tubes was added 100μl of the bead suspension, 500μl of the contrigate and 500μl of biotin solution. Each tube was capped and placed on an end-over-end mixer for 2 hours at ambient temperature.

After incubation, 50μl from each tube was removed and mixed with 1ml of buffer and placed in one of the gel-containing sample tubes. The beads immediately settled onto the gel surface, and after a few minutes a speckled blue colouration appeared in the tubes containing little or no free biotin. Very feint blue speckling could be seen in those tubes with higher levels of free biotin. As the reaction progressed, colour developed in all tubes, but more strongly and rapidly in the tubes with no free biotin. Better discrimination could be obtained by using a larger volume of diluent in the same system.

### Example 1b

A similar experiment can be performed using pH-sensitive paper in the tube cap, located inside a body of agarose gel containing urea as a releasable substrate, and with urease enzyme as the label on the biotin. Localised beads carrying bound biotin/urease will react with released urea, generating ammonia leading to a local pH change in the gel which is detected by the pH-sensitive paper. If the test sample is made up in a pH buffer, no pH change should occur in the volume of sample remote from the gel.

### Example 1c

In this Example, the same beads, conjugate and free biotin as in Example 1a above were used, but with a chemiluminescent detector gel.

The gel was based on 1% (w/v) agarose in 0.1M Tris buffer at pH8.5, containing 150mM glucose, 1.25mM luminol, 1mM para-iodophenol, and encapsulated HRP as in Example 1a. The HRP was included at a level of 1ml of capsules per 10ml gel, equivalent to 200 units of enzyme activity. Each sample tube contained 0.15ml of gel.

After incubation as in Example 1a, 50μl of beads bearing the glucose oxidase, in 2ml of PBS buffer, was added to each tube. The beads settled onto the gel surface, and in the case of samples which had contained little or no free biotin, a readily visible blue light was emitted by the gel layer.

### Claims

1. An assay method for determining qualitatively and/or quantitatively the presence of an analyte in a sample by means of specific binding, wherein the sample is contacted with a moveable solid phase carrier material, on which is immobilised a first binding reagent having specificity for the analyte and with a labelled specific binding reagent which can participate in either a "sandwich" or a "competition" reaction with the first reagent in the presence of the analyte, and in which method, following an incubation period sufficient to allow the reaction to take place, the moveable solid phase carrier material is moved within the assay medium to the immediate vicinity of a sensing means for the chemical "signal" and in the immediate vicinity of which sensing means a signal influencing agent is released into the assay medium, so enabling the generation of a detectable signal the nature and/or magnitude of which is dependent on the extent of binding that has taken place between the immunogenic species under test and the specific binding partner carried by the particles.

2. An assay method according to claim 1, wherein the signal influencing agent is a chemical entity that forms an essential part of the signal-generating chemical reaction.

3. An assay method according to claim 1, wherein the signal influencing agent is a chemical entity that enhances the efficiency of the chemical reaction by which the chemical "signal" is produced.

4. An assay method according to any one of the preceding claims, wherein incubation is conducted in a small volume of test sample and thereafter the sample is diluted prior to localisation of the carrier material in the vicinity of the sensing means.

5. An assay method according to any one of the preceding claims wherein the signal influencing agent diffuses from a source located on, in or near the signal sensing means.

6. An assay method according to any one of the preceding claims wherein the assay medium incorporates a masking or quenching agent which inhibits the production of observation of the chemical signal in regions remote from the signal sensing means.

7. An assay method according to any one of the preceding claims, wherein the signal detected by the sensing means is visible light originating from a chemiluminescent reaction.

8. An assay method according to claim 7, wherein the label generates hydrogen peroxide in the assay medium, and the peroxide is detected via its participation in a chemiluminescent reaction.

9. An assay method according to claim 7, wherein the label is a peroxide destroying enzyme which engages in a chemiluminescent reaction.

10. An assay method according to any one of claims 1 to 6, wherein the label generates hydrogen peroxide in the assay medium and the peroxide is measured electrochemically.

11. An assay method according to claim 6, wherein the detectable signal is chemiluminescent light and the masking or quenching reagent renders the assay medium opaque or at least insufficiently translucent for light from regions remote from the sensing means to reach the signal sensing means.

12. An assay method according to claim 6, wherein the masking or quenching reagent is a chemical reagent which consumes an essential component of the signal generating system.

13. An assay method according to claim 12, wherein the label produces hydrogen peroxide in the assay medium and the consumer reagent is catalase enzyme.

14. An assay method according to any one of claims 7 to 9, wherein the label is horesraddish peroxidase and the assay medium incorporates a peroxide, such as hydrogen peroxide, and a chemiluminescent 2,3-dihydro, 1,4-phthalazinedione, such as luminol, is released into the assay medium in the immediate vicinity of a light detection means.

15. An assay method according to any one of claims 7 to 9, wherein the label is glucose oxidase enzyme, the assay medium incorporates a chemiluminescent 2,3-dihydro, 1,4-phthalazinedione, such as luminol, and glucose is released into the assay medium in the immediate vicinity of a light detection means.

16. An assay method according to any one of claims 7 to 9, wherein the label is glucose oxidase enzyme, and the detection zone is a gel layer containing chemiluminescent 2,3-dihydro 1,4-phthalazinedione, such as luminol, bound horseraddish peroxidase enzyme, and glucose which is releasable into the assay medium to provide a substrate for the glucose oxidase enzyme.

17. An assay method according to claim 7, which utilises:

(a) a first population of localisable particles (e.g. "magnetic" particles) bearing a specific binding partner for a chemical species under test;

(b) a non-particle-borne specific binding partner labelled with a chemical reactant that can react with a substrate to produce a precursor for a chemiluminescent reaction;

(c) optionally, a consumer reagent dispersed in the assay medium which competes effectively for the precursor and can thereby inhibits the production of chemiluminescence while the labelled specific binding partner remains dispersed in the assay medium;

(d) a second population of localisable particles bearing or containing a reagent which can react with the precursor to generate chemiluminescence;

(e) a light detection means; and

(f) a source of substrate for the chemiluminescent reaction which can release the substrate into the medium in the immediate vicinity of the light detection means,

and in which assay co-localisation of the two particle populations adjacent the light detection means enables observation of chemiluminescence produced in an amount dependent on the extent to which the specific binding partner carried by the first population of particles has bound to the species under test.

18. An assay method according to claim 17, wherein the label is glucose oxidase reacting with glucose released into the assay medium in the immediate vicinity of the light detection means, and with oxygen in the assay medium to produce hydrogen peroxide as the precursor, the consumer is catalase enzyme, and the bound reagent is horseraddish peroxidase which interacts with luminol in the assay medium.

19. A device for use in a method according to claim 1 comprising a vessel in which the assay method can be conducted, incorporating a signal sensing means and a source of a signal-influencing agent located in, on or near the signal sensing means.

20. A device for use in a method according to claim 1, comprising an open-topped vessel for containing a volume of assay medium, the vessel being closeable by means of a cap within which is located a body of water-insoluble gel incorporating a signal-influencing agent which is releasable from the gel when the gel is contacted with an assay medium, the gel also containing such further reagents as may be necessary to enable a detectable signal to be generated.

21. A device according to claim 19 wherein the gel is aggarose gel.

22. An assay method utilising a device as claimed in claim 20 or claim 21, wherein an assay medium is prepared from:
    a) an aqueous sample;
    b) a particulate solid phase carrier material which can readily separate under gravity from an aqueous medium, the solid phase carrier material bearing an immobilised first binding reagent having specificity for an analyte suspected of being present in the sample; and
    c) a labelled specific binding reagent which can participate in either a "sandwich" or a "competition" reaction with the first reagent in the presence of the analyte; wherein a volume of the assay medium, either before or after an incubation period sufficient to allow the reaction to take place, is placed inside the device which is then closed with the cap and inverted to allow the particulate solid phase carrier material to settle onto the gel and enable an analytical result to be determined.

23. A method according to claim 22, wherein the label is glucose oxidase enzyme and the gel incorporates glucose which is releasable into the assay medium, together with potassium iodide, starch and horseradish peroxidase enzyme.

24. A method according to claim 22, wherein the label is glucose oxidase enzyme and the gel contains glucose which is releasable into the assay medium, together with luminol para-iodophenol and horseradish peroxidase enzyme.

_Fig. 1_

_Fig. 2_

0 270 206

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | EP-A-0 106 536  (ORTHO DIAGNOSTICS SYSTEMS INC.)<br>* figures 5a-e, claims 4, 5 *<br>--- | 1,2 | G 01 N  33/543 |
| A | EP-A-0 124 050  (PANDEX LABORATORIES INC.)<br>* page 10, line 24 - page 11, line 15; claims 1-8 *<br>--- | 1,7 | |
| A | EP-A-0 177 813  (LABSYSTEMS OY)<br>* page 2, line 31 - page 3, line 5; page 4, lines 6-10; figures 1-3; claims 1-10 *<br>--- | 1,6,11,<br>16,19 | |
| A | EP-A-0 178 790  (UNILEVER N.V.)<br>* claims 1-6, 10, 11, 13, 14 *<br>--- | 1-3,5-9<br>,12-18,<br>22-24 | |
| A | GB-A-2 049 185  (ORION-YHTYMA OY)<br>* figures 1, 2; claim 1 *<br>----- | 19 | |
| | | | TECHNICAL FIELDS SEARCHED (Int. Cl.4) |
| | | | G 01 N  33/00 |

The present search report has been drawn up for all claims

| Place of search | Date of completion of the search | Examiner |
|---|---|---|
| BERLIN | 20-10-1987 | DE KOK A.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................
& : member of the same patent family, corresponding document

EPO FORM 1503 03.82 (P0401)